# EUROPÄISCHE PATENTSCHRIFT

(11) **EP 3 321 350 B1**
(45) Veröffentlichungstag und Bekanntmachung des Hinweises auf die Patenterteilung: **02.09.2020**
(21) Anmeldenummer: 17200912.8
(22) Anmeldetag: 09.11.2017
(51) Int. Cl.: C12M 1/107, C12M 1/00, C12M 1/34

(54) **MEMBRANDACH**
MEMBRANE ROOF
TOITURE À MEMBRANE

(30) Priorität: 09.11.2016 DE 202016106267 U
(43) Veröffentlichungstag der Anmeldung: 16.05.2018
(73) Patentinhaber: JOPE Beteiligungs GmbH, 87653 Eggenthal (DE)
(72) Erfinder: Baur, Josef, 87784 Westerheim (DE); Baur, Peter, 87724 Ottobeuren (DE)
(74) Vertreter: Patentanwälte Olbricht, Buchold, Keulertz Partnerschaft mbB

(56) Entgegenhaltungen:
- EP-A1- 1 801 037
- EP-A2- 0 521 302
- WO-A1-2014/079401
- CH-A2- 704 818
- CH-A5- 628 130
- DE-A1- 2 212 892
- DE-A1-102008 003 755
- DE-A1-102013 012 707
- DE-A1-102013 114 715
- DE-A1-102013 210 140
- US-A1- 2015 299 632
- DATABASE WPI Week 200939 Thomson Scientific, London, GB; AN 2009-K06252 XP002779654, -& CN 201 241 138 Y (ANYANG AIERWANG ENVIRONMENT ENG CO LTD) 20. Mai 2009 (2009-05-20)

## Beschreibung

Die Erfindung betrifft ein Membrandach zur Abdeckung eines Behälters. Beispielsweise kann es sich bei einem solchen Behälter um einen Biomassereaktor, ein Becken oder eine Lagune handeln. Derartige Behälter erfordern typischerweise eine Abdeckung, welche das Entweichen von entstehendem Gas verhindert, da dieses Gas genutzt werden soll, beispielsweise zum Betrieb eines Blockheizkraftwerks.

CH 628 130 offenbart eine Methangasanlage mit einem flexiblen Foliendach, welches ein an unterschiedliche Drücke anpassbares Ausgleichselement auf seiner Oberfläche aufweist. Der Behälter des Ausgleichselements aus Dl hat ein konstantes Volumen zur variablen Füllung mit dem Beschwerungsmedium Sand..

Es hat sich jedoch gezeigt, dass aus dem Stand der Technik bekannte Membrandächer für manche Anwendungssituationen zu unflexibel sind, da sie sich nicht an unterschiedliche benötigte Volumina oder Drücke anpassen können, welche beispielsweise bei unterschiedlicher Gasproduktion, unterschiedlicher Entnahme oder unterschiedlicher Sonneneinstrahlung auftreten können.

Es ist deshalb eine Aufgabe der Erfindung, ein alternatives, insbesondere flexibler an unterschiedliche Drücke anpassbares Membrandach bereitzustellen. Es ist des Weiteren eine Aufgabe der Erfindung, einen Biomassereaktor mit einem solchen Membrandach bereitzustellen.

Dies wird erfindungsgemäß durch ein Membrandach gemäß Anspruch 1 und einen Biomassereaktor gemäß Anspruch 14 erreicht. Vorteilhafte Ausführungen können beispielsweise den jeweiligen Unteransprüchen entnommen werden.

Die Erfindung betrifft ein Membrandach zur Abdeckung eines Behälters, wie den Reaktionsraum eines Biomassereaktors, eines Beckens oder einer Lagune. Das Membrandach umfasst eine gasundurchlässige Dichtfolie, welche gasundurchlässig mit dem Rand des Behälters verbunden ist. Dies betrifft insbesondere einen Einbauzustand bzw. einen Zustand in welchem die Dichtfolie wie vorgesehen verbaut ist. Ansonsten kann auch davon gesprochen werden, dass die Dichtfolie mit dem Rand des Behälters verbindbar bzw. gasundurchlässig verbindbar ist.

Die Gasundurchlässigkeit kann sich dabei beispielsweise auf Luft, auf ein zu sammelndes Gas wie beispielsweise Biogas, das typischerweise insbesondere Methan enthält, oder auch auf ein beliebiges andere Gas oder ein beliebiges Gemisch von Gasen beziehen. Die Gasundurchlässigkeit kann sich auch auf alle Gase beziehen.

Der Verlauf der den Behälter überspannenden Dichtfolie bestimmt eine Dichtfläche. Dabei handelt es sich insbesondere um eine imaginäre Fläche, entlang welcher die Dichtfolie das Entweichen von Gas aus dem Behälter verhindert. Diese imaginäre Fläche wird dabei insbesondere durch die aktuelle Form der Dichtfolie bestimmt, welche von Gegebenheiten wie beispielsweise Gasdrücken und einwirkenden Kräften abhängen kann.

Die Dichtfolie umfasst zumindest ein Ausgleichselement, welches eine Wandung aus fluidundurchlässiger Folie aufweist, wobei die Wandung ein veränderliches Fluidvolumen fluidundurchlässig umschließt. Das Fluidvolumen ist über eine Füllöffnung im Ausgleichselement veränderbar, und das Ausgleichselement weist eine wirksame Abdeckfläche auf, welche stetig an die Dichtfläche anschließt. Auch die Abdeckfläche ist insbesondere eine imaginäre Fläche, oder eine Hilfsfläche, die durch die geometrische Gestaltung des Ausgleichselementes in Größe (zum Beispiel ein im Querschnitt kreisrundes, lang gestrecktes oder oval Ausgleichselemente) und Form bestimmt ist. Es kann somit beispielsweise die Dichtfläche partiell durch die wirksame Abdeckfläche fortgesetzt oder ersetzt werden, wobei auch bei der wirksamen Abdeckfläche die oben bereits erwähnte Gasundurchlässigkeit typischerweise gegeben ist.

Die wirksame Abdeckfläche nimmt mit einer Vergrößerung des Fluidvolumens ab und mit einer Verkleinerung des Fluidvolumens zu.

Durch diese Funktionalität ist es möglich, die typischerweise aus Dichtfläche und wirksamer Abdeckfläche gebildete Gesamtfläche, entlang welcher das Ausweichen von Gas aus dem Behälter verhindert wird, zu variieren. Damit kann unterschiedlichen Betriebsbedingungen, beispielsweise unterschiedlichen Drücken, Rechnung getragen werden.

Insbesondere kann dabei der Effekt ausgenutzt werden, dass ein kreisförmiger Querschnitt einer Wandung ein höheres Fluidvolumen einschließt als ein ovalförmiger oder länglicher Querschnitt der gleichen Wandung, letzterer aber eine größere Fläche abdeckt.

Gemäß einer bevorzugten Ausführung verändert, in einer Schnittansicht durch das Ausgleichselement betrachtet, eine Veränderung des Fluidvolumens des Ausgleichselementes die von der Wandung des Ausgleichselementes umschlossene Fläche. Das Fluidvolumen kann beispielsweise durch Zugeben oder Ablassen eines Fluids variiert werden.

Als Fluid kann beispielsweise eine Flüssigkeit, insbesondere Wasser oder Öl, oder auch ein Gas, insbesondere Luft, verwendet werden.

Die Dichtfolie ist bevorzugt im Wesentlichen nicht dehnbar. Sie kann auch vollständig nicht dehnbar sein. Damit kann das umschlossene Volumen vorteilhaft definiert werden.

Auch die Wandung ist bevorzugt im Wesentlichen nicht dehnbar und kann auch vollständig nicht dehnbar sein.

Gemäß einer typischen Ausführung dichtet das Membrandach ein in einem Behälter befindliches Gasvolumen gegenüber der Umgebung ab. Damit kann entstehendes Gas, beispielsweise Biogas, vorteilhaft gesammelt und verwendet werden.

Das Ausgleichselement kann insbesondere schlauchförmig ausgeführt sein. Dies entspricht einer einfachen Ausführung.

Bevorzugt befindet sich zumindest an einem Ende eines schlauchförmig ausgeführten Ausgleichselementes eine Füllöffnung. Dies erlaubt ein einfaches Befüllen oder auch Entleeren des Ausgleichselements.

Das Ausgleichselement kann vorteilhaft in der Mitte oder entlang einer Symmetrieachse des Membrandaches angeordnet sein. Dies hat sich als einfache Ausführung bewährt.

Das Ausgleichselement kann in der Draufsicht auf das Membrandach bei einem kreisrunden Membrandach ebenfalls kreisrund ausgeführt sein. Das Ausgleichselement kann auch in der Draufsicht auf das Membrandach bei einem rechteckigen Membrandach ebenfalls rechteckig ausgeführt sein.

An der Füllöffnung kann vorteilhaft eine Einfüllvorrichtung vorgesehen sein, welche das Fluidvolumen des Ausgleichselementes verändert. Die Einfüllvorrichtung kann zum Beispiel ein Gebläse oder eine Pumpe sein. Damit kann das Fluid in das Ausgleichselement eingeführt werden.

Das Ausgleichselement weist gemäß einer bevorzugten Ausführung ein Ablassventil auf, durch welches in dessen geöffnetem Zustand Fluid aus dem Ausgleichselement entweichen kann. Damit kann das Volumen des Ausgleichselements durch Ablassen des Fluids verkleinert werden.

In einer bevorzugten Ausgestaltung ist vorgesehen, dass das Material, aus welchen das Ausgleichselement besteht, identisch oder ähnlich ist dem Material der Dichtfolie. Dieser Materialvorschlag beinhaltet mehrere Vorteile. Zum einen ist das Verarbeiten von identischen oder ähnlichen Materialen einfach. Das Kunststofffolienmaterial, welches als Dichtfolie bzw. Ausgleichselement dient, kann zum Beispiel thermoplastische verschweißt werden. Eine einstoffige Ausgestaltung begünstigt des Weiteren auch das Verwerten eines nicht mehr benötigten Membrandaches. Ein aufwändiges Trennen der unterschiedlichen Materialien ist nicht notwendig. Als ähnliche Materialien werden dabei Materialen angesehen, die in ihrer chemischen und/oder physikalischen Eigenschaften einander ähneln, zum Beispiel in gleicher Weise verarbeitet oder aufgearbeitet bzw. verwertet werden können.

Das Ablassventil kann beispielsweise durch eine mechanische, insbesondere keine elektrische Energie benötigende Steuervorrichtung betätigbar sein. Alternativ kann das Ablassventil beispielsweise auch durch ein elektronisch geregeltes Stellglied betätigbar sein.

Das Membrandach kann einen Sensor aufweisen, der auf oder in der Dichtfolie angebracht ist und der die in der Dichtfolie herrschende mechanische Spannung oder den im Behälter, unter der Dichtfolie herrschenden Gasdruck misst.

Das Membrandach kann auch einen Nahtsensor aufweisen, welcher am Rand des Membrandaches, an der Grenze zwischen Membrandach und dem von dem Membrandach abgedeckten Behälter angebracht ist und der die mechanische Spannung der Verbindung zwischen Membrandach und Behälter misst.

Mittels der Sensoren kann eine Druckregelung und/oder Spannungsregelung implementiert werden, welche beispielsweise über das Ausgleichselement funktionieren kann. Beispielsweise kann durch Einbringen oder Ablassen von Fluid das Volumen so angepasst werden, dass ein vorgegebener Druck erreicht wird.

Gemäß einer Weiterbildung können mehrere, insbesondere parallel zueinander angeordnete, schlauchförmige Ausgleichselemente vorgesehen sein. Damit kann die oben beschriebene Wirkung eines Ausgleichselements vervielfacht werden. Die Ausgleichselemente können beispielsweise getrennt oder auch zusammen angesteuert werden. Dementsprechend können sie getrennte Volumina oder auch ein gemeinsames Volumen ausbilden.

Für jedes einzelne Ausgleichselement sind die hierin offenbarten Ausführungen und Varianten entsprechend anwendbar.

Die durch eine Veränderung des Fluidvolumens erzeugte Änderung der wirksamen Abdeckfläche mehrerer Ausgleichselemente kann sich insbesondere addieren.

Zumindest ein Ausgleichselement kann sich gemäß einer Ausführung am Rand des Membrandaches befinden.

Das Ausgleichselement kann insbesondere mit einem Fluid, z.B. einem Gas, wie z.B. Luft oder einer Flüssigkeit, wie z.B. Wasser befüllbar sein. Dies hat sich bewährt, jedoch können grundsätzlich auch andere Fluide verwendet werden.

Das am Rand des Membrandaches im Bereich des Randes des Behälters angeordnete oder hierzu beabstandete Ausgleichselement kann insbesondere mit einer Flüssigkeit befüllbar sein.

Es kann gemäß einer Ausführung auch eine Steuerung vorgesehen sein, welche über zumindest einen Sensor die mechanische Spannung des Membrandaches unmittelbar oder mittelbar erfasst und basierend auf dieser gemessenen Spannung das Fluidvolumen zumindest eines Ausgleichselementes regelt. Es kann auch eine mittelbare Erfassung z.B. über den Druck im Druckraum erfolgen.

Die Erfindung betrifft des Weiteren einen Biomassereaktor mit einem Behälter, z.B. dem Reaktionsraum eines Biomassereaktors, und einem erfindungsgemäßen Membrandach, das den Behälter gasdicht verschließt. Damit können die weiter oben beschriebenen Vorteile für einen Biomassereaktor nutzbar gemacht werden. Hinsichtlich des Membrandachs kann auf alle beschriebenen Ausführungen und Varianten zurückgegriffen werden.

Vorteilhaft kann sich im Inneren des Biomassereaktors ein Sensor zur Bestimmung des Gasdruckes befinden. Damit können die weiter oben bereits beschriebenen Vorteile und Funktionalitäten erreicht werden.

In der Zeichnung ist die Erfindung insbesondere in einem Ausführungsbeispiel schematisch dargestellt. Es zeigen:
- Fig. 1: ein Membrandach gemäß der Erfindung mit einer Dichtfolie in einer perspektivischen Ansicht,
- Fig. 2: das Membrandach gemäß der Erfindung in einer seitlichen Schnittansicht,
- Fig. 3: drei Zustände eines Ausgleichselements nach der Erfindung , und
- Fig. 4: ein Membrandach gemäß der Erfindung mit zwei Ausgleichselementen.

In den Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

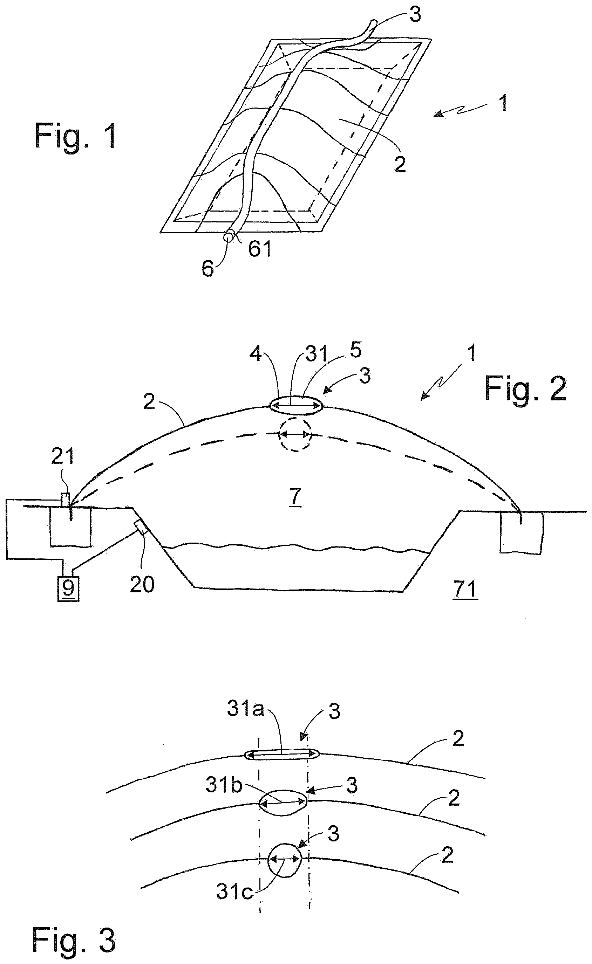

Fig. 1 zeigt ein Membrandach 1 mit einer Dichtfolie 2 gemäß einem Ausführungsbeispiel der Erfindung in einer perspektivischen Ansicht. Die Dichtfolie 2 deckt ein darunter befindliches Gasvolumen ab, auf welches weiter unten näher eingegangen werden wird.

An der Dichtfolie 2 ist ein Ausgleichselement 3 angebracht. Dieses legt sich vorliegend wie gezeigt schlauchförmig über die Dichtfolie 2 bzw. ist darin eingearbeitet.

Endseitig an dem Ausgleichselement 3 ist eine Füllöffnung 6 angeordnet. Mittels dieser Füllöffnung 6 kann ein Fluid in das Ausgleichselement 3 eingebracht werden. Hierzu befindet sich an der Füllöffnung 6 eine Einfüllvorrichtung 61 in Form einer Pumpe, welche lediglich schematisch dargestellt ist.

Fig. 2 zeigt das Membrandach 1 von Fig. 1 in einer seitlichen Schnittansicht. Dabei ist auch das Ausgleichselement 3 zu erkennen.

Das Ausgleichselement 3 weist eine Wandung 4 auf. Diese umschließt ein Fluidvolumen 5 innerhalb des Ausgleichselements 3.

Die Dichtfolie 2 deckt einen Behälter 71 ab, wozu sie randseitig mit dem Behälter 71 gasdicht verbunden ist. Dadurch entsteht ein Gasvolumen 7, welches von der Dichtfolie 2 nach oben begrenzt wird. Die Dichtfolie 2 ist hierzu gasundurchlässig ausgebildet, so dass das gesamte im Gasvolumen 7 befindliche Gas am Entweichen gehindert wird.

In Fig. 2 sind zwei Zustände des Ausgleichselements 3 dargestellt, einer oben in durchgezogener Linie und einer unten in gestrichelter Linie.

In dem oben dargestellten Zustand hat das Ausgleichselement 3 eine ovale Form und ist in horizontaler Richtung langgestreckt. Dies entspricht einem Zustand, in welchem nur wenig Fluidvolumen 5 im Ausgleichselement 3 vorhanden ist. Dabei wird eine größere wirksame Abdeckfläche 31 überspannt, welche zusammen mit der Dichtfolie 2 das Gasvolumen 7 begrenzt. Hierdurch kann auf einen Druckanstieg in dem von dem Membrandach überspanntem Volumen reagiert werden. Gegebenenfalls ist auch ein (Druck- und/oder Temperatur-) Sensor mit einer Steuerung verbunden, die auf ein Gebläse oder Pumpe zum Verändern des Fluidvolumens wirkt.

Wird hingegen zusätzliches Fluid in das Ausgleichselement 3 eingeführt, was beispielsweise mittels der Pumpe 61 erfolgen kann, so vergrößert sich das Fluidvolumen 5 und es kann der unten dargestellte Zustand eingenommen werden. Dabei hat das Ausgleichselement 3 in etwa einen kreisförmigen Querschnitt. Die wirksame Abdeckfläche 31 ist dementsprechend kleiner, was auch insgesamt dazu führt, dass die Dichtfolie 2 tiefer gespannt ist. Damit wird ein kleineres Gasvolumen 7 abgedeckt.

Zur Überwachung des Gasdrucks im Gasvolumen 7 ist ein Gassensor 20 vorgesehen. Des Weiteren ist zur Überwachung der Spannung an einer Verbindung zwischen Behälter 71 und Dichtfolie 2 ein Nahtsensor 21 vorgesehen. Die von den beiden Sensoren 20, 21 gelieferten Signale werden von einer Steuerung 9 ausgewertet und dementsprechend wird die Einfüllvorrichtung 61 gesteuert, so dass ein gewünschter vorgegebener Gasdruck aufrecht erhalten wird und die Verbindung zwischen Dichtfolie 2 und Behälter 71 nicht überbeansprucht wird.

In Fig. 3 sind drei unterschiedliche Zustände des Ausgleichselements 3 im Querschnitt dargestellt. Im obersten Zustand ist das Ausgleichselement 3 horizontal langestreckt und überspannt eine weite wirksame Abdeckfläche 31a. Dies entspricht einem besonders kleinen Fluidvolumen 5. Im mittleren Zustand ist das Fluidvolumen 5 etwas größer, die Form ellipsenförmig und die wirksame Abdeckfläche 31b ist etwas kleiner. Im untersten Zustand ist das Fluidvolumen 5 noch größer, die Form kreisförmig und die wirksame Abdeckfläche 31c ist am kleinsten. An diesem Beispiel wird die Funktionsweise der Erfindung ganz besonders deutlich. Die (gedachte, imaginäre) Abdeckfläche31a, 31b, 31c ist durch die Form des Ausgleichselements 3 in Größe und Form definiert, und schließt stetig an die Dichtfläche, um so die Funktionsweise der Erfindung zu beschreiben.

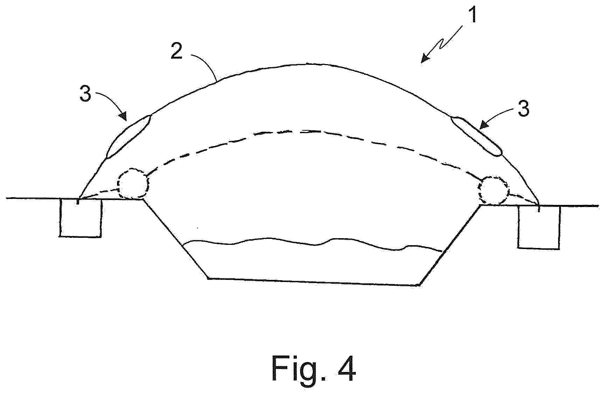

In Fig. 4 ist schematisch ein zweites Ausführungsbeispiel eines Membrandachs 1 gezeigt, welches im Vergleich zum ersten Ausführungsbeispiel nicht nur ein Ausgleichselement 3, sondern zwei Ausgleichselemente 3 aufweist. Diese können sowohl gemeinsam wie auch getrennt voneinander angesteuert werden und bieten jeweils die weiter oben beschriebene Funktionalität.

Auch ein weiterer Zustand ist in Fig. 4 gezeigt, welcher mit gestrichelten Linien dargestellt ist. Ähnlich wie bei Fig. 2 entspricht dieser einem höheren Fluidvolumen 5 und damit entsprechend geringerer wirksamer Abdeckfläche 31.

Ansonsten sei bezüglich der Ausgestaltung der beiden Ausgleichselemente 3 der Ausführung nach Fig. 4 auf die Beschreibung der Figuren 1 bis 3 verwiesen.

Die jetzt mit der Anmeldung und später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Membrandach zur Abdeckung eines Behälters, wie den Reaktionsraum eines Biomassereaktors, eines Beckens oder einer Lagune, umfassend eine gasundurchlässige Dichtfolie (2) welche gasundurchlässig mit dem Rand des Behälters verbunden ist und der Verlauf der den Behälter überspannenden Dichtfolie (2) eine Dichtfläche bestimmt und die Dichtfolie zumindest ein Ausgleichselement (3) umfasst, welches eine Wandung (4) aus fluidundurchlässiger Folie aufweist und wobei die Wandung (4) ein veränderliches Fluidvolumen (5) fluidundurchlässig umschließt, wobei das Fluidvolumen (5) über eine Füllöffnung (6) im Ausgleichselement (3) veränderbar ist, und das Ausgleichselement (3) eine wirksame Abdeckfläche (31) aufweist, welche stetig an die Dichtfläche anschließt und wobei die wirksame Abdeckfläche (31) mit einer Vergrößerung des Fluidvolumens (5) abnimmt und mit einer Verkleinerung des Fluidvolumens (5) zunimmt.

2. Membrandach nach Anspruch 1, **dadurch gekennzeichnet, dass** in einer Schnittansicht durch das Ausgleichselement (3) betrachtet, eine Veränderung des Fluidvolumens (5) des Ausgleichselementes (3), die von der Wandung (4) des Ausgleichselementes (3) umschlossene Fläche verändert und/oder das Ausgleichselement (3) schlauchförmig ausgeführt ist.

3. Membrandach nach einem der vorhergehenden Ansprüche, **dadurch gekennzeichnet, dass** die Dichtfolie (2) im Wesentlichen nicht dehnbar ist.

4. Membrandach nach einem der vorhergehenden Ansprüche, **dadurch gekennzeichnet, dass** das Membrandach ein, in einem Behälter befindliches Gasvolumen (7) gegenüber der Umgebung abdichtet.

5. Membrandach nach einem der vorhergehenden Ansprüche, **dadurch gekennzeichnet, dass** sich zumindest an einem Ende eines bevorzugt schlauchförmig ausgeführten Ausgleichselementes (3) eine Füllöffnung (6) befindet und/oder das Ausgleichselement (3) in der Mitte oder entlang einer Symmetrieachse des Membrandaches angeordnet ist.

6. Membrandach nach einem der vorhergehenden Ansprüche, **dadurch gekennzeichnet, dass** das Ausgleichselement (3) in der Draufsicht auf das Membrandach bei einem kreisrunden Membrandach ebenfalls kreisrund ausgeführt ist, oder das Ausgleichselement (3) in der Draufsicht auf das Membrandach bei einem rechteckigen Membrandach ebenfalls rechteckig ausgeführt ist.

7. Membrandach nach einem der vorhergehenden Ansprüche, **dadurch gekennzeichnet, dass** an der Füllöffnung (6) eine Einfüllvorrichtung (61) vorgesehen ist, welche das Fluidvolumen (5) des Ausgleichselementes (3) verändert und/oder das Ausgleichselement (3) ein Ablassventil (8) aufweist, durch welches in dessen geöffnetem Zustand Fluid aus dem Ausgleichselement (3) entweichen kann.

8. Membrandach nach einem der vorhergehenden Ansprüche, **dadurch gekennzeichnet, dass** das Ablassventil (8) durch eine mechanische, insbesondere keine elektrische Energie benötigende Steuervorrichtung betätigbar ist und/oder das Ablassventil (8) durch ein elektronisch geregeltes Stellglied betätigbar ist.

9. Membrandach nach einem der vorhergehenden Ansprüche, **dadurch gekennzeichnet, dass** das Membrandach einen Sensor (20) aufweist, der auf oder in der Dichtfolie (2) angebracht ist und der die in der Dichtfolie (2) herrschende mechanische Spannung oder den im Behälter, unter der Dichtfolie herrschenden Gasdruck misst und/oder das Membrandach einen Nahtsensor (21) aufweist, welcher am Rand des Membrandaches, an der Grenze zwischen Membrandach und dem von dem Membrandach abgedeckten Behälter angebracht ist und der die mechanische Spannung der Verbindung zwischen Membrandach und Behälter misst.

10. Membrandach nach einem der vorhergehenden Ansprüche, **dadurch gekennzeichnet, dass** mehrere, insbesondere parallel zueinander angeordnete, schlauchförmige Ausgleichselemente (3) vorgesehen sind und/oder dass das Material, aus welchen das Ausgleichselement (3) besteht, identisch oder ähnlich ist dem Material der Dichtfolie (2).

11. Membrandach nach einem der vorhergehenden Ansprüche, **dadurch gekennzeichnet, dass** sich die durch eine Veränderung des Fluidvolumens (5) erzeugte Änderung der wirksamen Abdeckfläche (31) mehrerer Ausgleichselemente (3) addiert und/oder sich zumindest ein Ausgleichselement (3) am Rand des Membrandaches befindet und/oder das Ausgleichselement (3) mit einem Fluid, z.B. einem Gas, wie z.B. Luft oder einer Flüssigkeit, wie z.B. Wasser befüllbar ist.

12. Membrandach nach einem der vorhergehenden Ansprüche, **dadurch gekennzeichnet, dass** das am Rand des Membrandaches im Bereich des Randes des Behälters, angeordnete oder hierzu beabstandete Ausgleichselement (3) mit einer Flüssigkeit befüllbar ist.

13. Membrandach nach einem der vorhergehenden Ansprüche, **dadurch gekennzeichnet, dass** eine Steuerung (9) vorgesehen ist, welche über zumindest einen Sensor die mechanische Spannung des Membrandaches unmittelbar oder mittelbar erfasst und basierend auf dieser gemessenen Spannung das Fluidvolumen (5) zumindest eines Ausgleichselementes (3) regelt.

14. Biomassereaktor mit einem Behälter, z.B. den Reaktionsraum eines Biomassereaktors, und einem Membrandach nach einem der vorhergehenden Ansprüche, das den Behälter gasdicht verschließt.

15. Biomassereaktor nach Anspruch 14, **dadurch gekennzeichnet, dass** sich im Inneren des Biomassereaktors ein Sensor zur Bestimmung des Gasdruckes befindet.

## Claims

1. Membrane roof for covering a container, such as the reaction chamber of a biomass reactor, a tank or a lagoon, comprising a gas-impermeable sealing film (2) which is joined to the edge of the container in a gas-impermeable manner and the course of the sealing film (2) spanning the container determines a sealing surface and the sealing film comprises at least one compensating element (3) which has a wall (4) consisting of a fluid-impermeable film, and wherein the wall (4) surrounds a variable fluid volume (5) in a fluid-impermeable manner, wherein the fluid volume (5) can be varied via a filling opening (6) in the compensating element (3), and the compensating element (3) has an effective cover surface (31), which is continuously attached to the sealing surface and wherein the effective cover surface (31) decreases with an increase of the fluid volume (5) and increases with a reduction of the fluid volume (5).

2. Membrane roof according to claim 1, **characterised in that** as viewed in a cross-sectional view of the compensating element (3), there is a change in the fluid volume (5) of the compensating element (3), which changes the area surrounded by the wall (4) of the compensating element (3) and/or the compensating element (3) is designed to be hose-like.

3. Membrane roof according to any of the preceding claims, **characterised in that** the sealing film (2) is essentially not stretchable.

4. Membrane roof according to any of the preceding claims, **characterised in that** the membrane roof seals a volume of gas (7) located in a container from the environment.

5. Membrane roof according to any of the preceding claims, **characterised in that** at least at one end of a preferably hose-like compensating element (3) there is a filling opening (6) and/or the compensating element (3) is arranged in the centre or along an axis of symmetry of the membrane roof.

6. Membrane roof according to any of the preceding claims, **characterised in that** the compensating element (3) in plan view of the membrane roof with a circular membrane roof is also designed to be circular or the compensating element (3) in plan view of the membrane roof with a rectangular membrane roof is also designed to be rectangular.

7. Membrane roof according to any of the preceding claims, **characterised in that** at the filling opening (6) a filling device (61) is provided, which changes the fluid volume (5) of the compensating element (3) and/or the compensating element (3) has a discharge valve (8), through which in this opened state fluid can escape from the compensating element (3).

8. Membrane roof according to any of the preceding claims, **characterised in that** the discharge valve (8) can be activated by a mechanical control device, in particular one that does not require electrical power, and/or the discharge valve (8) can be activated by an electronically controlled actuator.

9. Membrane roof according to any of the preceding claims, **characterised in that** the membrane roof has a sensor (20), which is arranged on or in the sealing film (2) and which measures the mechanical tension in the sealing film (2) or the gas pressure in the container below the sealing film and/or the membrane roof has a seam sensor (21), which is attached onto the edge of the membrane roof, at the boundary between the membrane roof and the container covered by the membrane roof and which measures the mechanical tension of the connection between the membrane roof and container.

10. Membrane roof according to any of the preceding claims, **characterised in that** a plurality of hose-like compensating elements (3), arranged in particular parallel to one another, are provided and/or **in that** the material, from which the compensating element (3) is made, is identical or similar to the material of the sealing film (2).

11. Membrane roof according to any of the preceding claims, **characterised in that** the change in the effective cover surface (31) produced by a change in the fluid volume (5) of a plurality of compensating elements (3) is added up and/or at least one compensating element (3) is located at the edge of the membrane roof and/or the compensating element (3) can be filled with a fluid, e.g. a gas, such as e.g. air or a fluid, such as e.g. water.

12. Membrane roof according to any of the preceding claims, **characterised in that** the compensating element (3) arranged at the edge of the membrane roof in the region of the edge of the container, or spaced apart from the latter, can be filled with a fluid.

13. Membrane roof according to any of the preceding claims, **characterised in that** a controller (9) is provided, which by means of at least one sensor detects the mechanical tension of the membrane roof directly or indirectly and based on this measured tension the fluid volume (5) controls at least one compensating element (3).

14. Biomass reactor with a container, e.g. the reaction chamber of a biomass reactor, and a membrane roof according to any of the preceding claims, which closes the container in a gastight manner.

15. Biomass reactor according to claim 14, **characterised in that** inside the biomass reactor there is a sensor for determining the gas pressure.

## Revendications

1. Toiture à membrane destinée à recouvrir un récipient, comme la chambre de réaction d'un réacteur à biomasse, d'un bassin ou d'un lagon, comprenant un film d'étanchéité imperméable au gaz (2) lequel est connecté de manière imperméable au gaz au bord du récipient et le tracé du film d'étanchéité (2) enjambant le récipient détermine une surface d'étanchéité et le film d'étanchéité présente au moins un élément de compensation (3), lequel présente une paroi (4) en film imperméable au fluide et dans lequel la paroi (4) renferme un volume de fluide (5) variable de manière imperméable au fluide, dans lequel le volume de fluide (5) peut être modifié via une ouverture de remplissage (6) dans l'élément de compensation (3), et l'élément de compensation (3) présente une surface de recouvrement (31) effective, laquelle se raccorde de façon constante sur la surface d'étanchéité et dans lequel la surface de recouvrement (31) effective diminue avec une augmentation du volume de fluide (5) et augmente avec une diminution du volume de fluide (5).

2. Toiture à membrane selon la revendication 1, **caractérisée en ce que** lorsqu'elle est vue dans une vue en coupe par l'élément de compensation (3), une modification du volume de fluide (5) de l'élément de compensation (3), qui modifie une surface renfermée par la paroi (4) de l'élément de compensation (3) et/ou l'élément de compensation (3) est réalisé en forme de tube.

3. Toiture à membrane selon l'une quelconque des revendications précédentes, **caractérisée en ce que** le film d'étanchéité (2) est essentiellement non extensible.

4. Toiture à membrane selon l'une quelconque des revendications précédentes, **caractérisée en ce que** la toiture à membrane rend un volume de gaz (7) se trouvant dans un récipient étanche par rapport à l'environnement.

5. Toiture à membrane selon l'une quelconque des revendications précédentes, **caractérisée en ce qu'une** ouverture de remplissage (6) se trouve à au moins une extrémité d'un élément de compensation (3) exécuté de préférence en forme de tube et/ou l'élément de compensation (3) est agencé au centre ou le long d'un axe de symétrie de la toiture à membrane.

6. Toiture à membrane selon l'une quelconque des revendications précédentes, **caractérisée en ce que** l'élément de compensation (3) dans la vue du dessus est également exécuté de manière circulaire sur la toiture à membrane dans le cas d'une toiture à membrane circulaire, ou **en ce que** l'élément de compensation (3) dans la vue du dessus est également rectangulaire sur la toiture à membrane dans le cas d'une toiture à membrane rectangulaire.

7. Toiture à membrane selon l'une quelconque des revendications précédentes, **caractérisée en ce qu'**un dispositif de remplissage (61) est prévu à l'ouverture de remplissage (6), laquelle modifie le volume de fluide (5) de l'élément de compensation (3) et/ou l'élément de compensation (3) présente une vanne de décharge (8), à travers laquelle un fluide peut être évacué de l'élément de compensation (3) dans son état ouvert.

8. Toiture à membrane selon l'une quelconque des revendications précédentes, **caractérisée en ce que** la vanne de décharge (8) peut être actionnée par l'intermédiaire d'un dispositif de commande mécanique, en particulier sans énergie électrique, et/ou la vanne de décharge (8) peut être actionnée par un actionneur à commande électronique.

9. Toiture à membrane selon l'une quelconque des revendications précédentes, **caractérisée en ce que** la toiture à membrane présente un capteur (20) qui est apposé sur ou dans le film d'étanchéité (2) et qui mesure la tension mécanique régnant dans le film d'étanchéité (2) ou la pression du gaz régnant sous le film d'étanchéité dans le récipient et/ou la toiture à membrane présente un capteur de couture (21) qui est apposé sur le bord de la toiture à membrane, à la limite entre la toiture à membrane et le récipient recouvert par la toiture à membrane et qui mesure la tension mécanique de la connexion entre la toiture à membrane et le récipient.

10. Toiture à membrane selon l'une quelconque des revendications précédentes, **caractérisée en ce que** plusieurs éléments de compensation (3) en forme de tubes, en particulier agencés de manière parallèle l'un par rapport à l'autre, sont prévus et/ou **en ce que** le matériau dont l'élément de compensation (3) est constitué est identique ou similaire au matériau du film d'étanchéité (2).

11. Toiture à membrane selon l'une quelconque des revendications précédentes, **caractérisée en ce que** le changement de la surface de couverture (31) effective de plusieurs éléments de compensation (3) produit par une modification du volume de fluide (5) s'ajoute et/ou au moins un élément de compensation (3) se trouve sur le bord de la toiture à membrane et/ou l'élément de compensation (3) peut être rempli avec un fluide, par exemple un gaz, comme l'air ou un liquide, par exemple de l'eau.

12. Toiture à membrane selon l'une quelconque des revendications précédentes, **caractérisée en ce que** l'élément de compensation (3), agencé sur le bord de la toiture à membrane dans la zone du bord du récipient ou espacé à cette fin, peut être rempli avec un liquide.

13. Toiture à membrane selon l'une quelconque des revendications précédentes, **caractérisée en ce qu'**une commande (9) est prévue, laquelle saisit directement ou indirectement, via au moins un capteur, la tension mécanique de la toiture à membrane et régule, sur la base de cette tension mesurée, le volume de fluide (5) d'au moins un élément de compensation (3).

14. Réacteur à biomasse avec un récipient, par exemple la salle de réaction d'un réacteur à biomasse, et une toiture à membrane selon l'une quelconque des revendications précédentes, qui ferme le récipient de manière étanche au gaz.

15. Réacteur à biomasse selon la revendication 14, **caractérisé en ce qu'**un capteur se trouve à l'intérieur du réacteur à biomasse pour déterminer la pression du gaz.
